# EUROPEAN PATENT APPLICATION

(11) **EP 1 413 292 A1**
(43) Date of publication of application: **28.04.2004**
(21) Application number: 03022770.6
(22) Date of filing: 10.10.2003
(51) Int. Cl.: A61K 9/00

(54) **Bioadhesive pharmaceutical compositions based on non-steroidal anti-inflammatory drugs**

(30) Priority: 25.10.2002 IT mi20022271
(71) Applicant: FARMAKA S.r.l., I-20123 Milano (IT)
(72) Inventor: Valenti, Mauro, 22070 Grandate (CO) (IT); Fornara, Carlo, 22070 Grandate (CO) (IT)
(74) Representative: Minoja, Fabrizio, Dr.

(57) **Abstract**

Disclosed are bioadhesive pharmaceutical compositions in the form of viscosified solutions or suspensions containing non-steroidal antiinflammatory drugs, polyvinylpyrrolidone and polyacrylic acids cross-linked with divinyl glycol.

## Description

The present invention relates to bioadhesive pharmaceutical compositions in the form of viscosified solutions containing non-steroidal anti-inflammatory drugs, polyvinylpyrrolidone and polyacrylic acids cross-linked with divinyl glycol.

The compositions according to the invention are particularly useful in the treatment of inflammatory states of the oral cavity.

Non-steroidal anti-inflammatory drugs which can be advantageously formulated according to the invention include arylpropionic acids (ibuprofen, ketoprofen, naproxen, flurbiprofen, etc.), oxicams (piroxicam and meloxicam), phenylacetic acids (diclofenac, alclofenac, sulindac and fenclofenac), and salicylic derivatives (diflunisal and acetylsalicylic acid).

The class of phenylacetic acids, especially diclofenac, possibly salified with choline (EP 521393) or with other pharmaceutically acceptable cations, is preferred.

Diclofenac is a pharmacologically active substance commonly used in the pharmaceutical field, because of its anti-inflammatory characteristics, in the form of solid oral, injectable, and transdermal topical formulations.

Liquid forms for the treatment of painful inflammatory states of the oral cavity were also recently marketed, in particular for the local treatment of irritative states of the oropharyngeal cavity associated with pain, such as gingivitis, stomatitis and pharyngitis, including those resulting from conservative or extractive dental surgery.

For this purpose, the drug is applied locally several times a day by means of gargling or mouthwashes.

The amount of drug which remains on the oral mucosa concerned is thus relatively small, and the resulting systemic action is very mild.

It has now been found that the pharmaceutical compositions of the invention, suitably viscosified and made bioadhesive with suitable polymers, enable the drug to remain in situ for a longer time and prolong its therapeutic action, so that fewer administrations are required.

The viscosified aqueous solutions or suspensions according to the invention form an adhesive film on the mucous membranes of the oropharyngeal cavity (mouth and throat), thus ensuring greater persistence of the drug at the point where the pharmacological action is required.

The characteristics of the adhesive film are crucial to the therapeutic effect and, in case of formulations designed to treat the oral mucosa, must meet a series of specific requirements for buccal administration (stability in contact with saliva, organoleptic characteristics, and absence of colouring or damaging effects on the tooth enamel) which are hard to achieve with the viscosifying and bioadhesive agents conventionally used in formulations designed for topical administration in other areas, such as carbomer, polycarbophil, poloxamers, methyl and hydroxypropyl cellulose ethers, polyvinyl alcohol, natural rubbers such as carrageenan and the like.

It has now been surprisingly found, and this is the object of the present invention that the most marked and advantageous film-forming effect is achieved by combining polyvinylpyrrolidone and polyacrylic acids cross-linked with divinyl glycol (polycarbophil).

Polyvinylpyrrolidone or povidone (PVP) is an excipient commonly used in the pharmaceutical field as a binding, suspending and complexing agent to improve the solubility of some active ingredients which are poorly soluble in water. These actions are closely dependent on the molecular weight of the polymer. In this specific case it is preferable to use PVP with an average to high molecular weight (K25 - K120), in an amount of between approx. 1% and approx. 20% on the weight of the total formulation.

Polycarbophil is a polymer constituted by units of polyacrylic acid cross-linked with divinyl glycol which gives the product a lattice structure able to trap water and form more or less viscous gels, in which molecules of the active ingredient are also trapped. The amount of polycarbophil used in the formulations according to the invention is generally between 0.1 % and 2% by weight.

The concentration of non-steroidal anti-inflammatory drug can vary within a wide range, depending on the specific compound selected. Broadly speaking, these concentrations will in any event be between 0.01 and 5% by weight.

In the case of diclofenac, preferably in the form of choline salt or any other salified form currently used in the pharmaceutical field (sodium salt, potassium salt or the like), the concentration will usually be between 0.01% and 1% by weight.

The compositions according to the invention can also contain other excipients commonly used to formulate liquid oral forms such as sweeteners, surfactants, flavourings, preservatives, antioxidants, etc.

Examples of said excipients include:
**sweeteners:** saccharose, xylitol, fructose, sorbitol, mannitol, hydrogenated glucose syrup, aspartame, acesulfame and saccharine;
**synthetic or natural antimicrobial agents:** esters of benzoic acid and its salts, benzoic acid, sorbic acid, chlorhexidine, benzalkonium chloride, cetylpyridinium chloride and grapefruit seed extracts;
**antioxidants:** butylhydroxyanisol (BHA), butylhydroxytoluene (BHT), ascorbic acid, sodium metabisulphite, lipoic acid and gallic acid esters;
**surfactants:** polyethoxylated hydrogenated castor oil, sorbitol esters (polysorbates), etc.

The following examples illustrate the invention in greater detail.

### Example 1

### Composition for 100 ml of mouthwash

| **INGREDIENTS** | **AMOUNT** |
|---|---|
| DICLOFENAC ACID | 0.074 g |
| CHOLINE BASE 50% | q.s. to pH 7.0-7.5 |
| POLYCARBOPHIL AA1 | 0.1500 g |
| POLYVINYLPYRROLIDONE K90 | 1.000 g |
| XYLITOL | 15.000g |
| SODIUM SACCHARINATE | 0.150g |
| SODIUM EDETATE | 0.025 g |
| SODIUM BENZOATE | 0.500 g |
| GLYCERIN | 5.000 g |
| PROPYLENE GLYCOL | 5.000 g |
| MINT FLAVOURING | 0.150g |
| POLYOXYETHYLENATED HYDROGENATED (40) CASTOR OIL | 0.700g |
| PONCEAU RED COLOURING E124 | 0.005 g |
| PURIFIED WATER | q.s. to 100 ml |

### Preparation method

Xylitol, glycerin, propylene glycol, sodium saccharinate, sodium edetate, sodium benzoate, polycarbophil AA1, PVP k90 and the colouring are solubilised (in that order) in approx. two-thirds of the purified water.

The mint flavouring, choline base and diclofenac are solubilised separately in the hydrogenated POE castor oil.

The solution of the active ingredient is added to the main solution, stirring until a homogeneous solution is obtained, pH is adjusted with the 50% choline base solution and make up to final volume.

Finally, stirring is continued until the solution is completely homogenous.

### Example 2

A composition with the following composition is prepared, according to the procedure of Example 1:

| **INGREDIENTS** | **AMOUNT** |
|---|---|
| DICLOFENAC ACID | 0.074g |
| CHOLINE BASE 50% | q.s. to pH 7.0-7.5 |
| POLYCARBOPHIL (NOVEON AA1) | 0.225 g |
| POLYVINYLPYRROLIDONE K90 | 1.500 g |
| XYLITOL | 15.000 g |
| SODIUM SACCHARINATE | 0.150 g |
| SODIUM EDETATE | 0.025g |
| SODIUM BENZOATE | 0.500g |
| GLYCERIN | 5.000 g |
| PROPYLENE GLYCOL | 5.000 g |
| MINT FLAVOURING | 0.150g |
| HYDROGENATED (40) POLYOXYETHYLENATED CASTOR OIL | 0.700 g |
| PONCEAU RED COLOURING E 124 | 0.005 g |
| PURIFIED WATER | q.s. to 100 ml |

### Example 3

A composition with the following composition is prepared, according to the procedure of Example 1:

| **INGREDIENTS** | **AMOUNT** |
|---|---|
| DICLOFENAC ACID | 0.074 g |
| SODIUM BICARBONATE | q.s. to pH 7.0-7.5 |
| POLYCARBOPHIL AA1 | 0.1500g |
| POLYVINYLPYRROLIDONE K90 | 1.000g |
| XYLITOL | 10.000 g |
| SORBITOL | 5.000 g |
| SODIUM SACCHARINATE | 0.150g |
| SODIUM EDETATE | 0.025g |
| SODIUM BENZOATE | 0.500g |
| GLYCERIN | 5.000 g |
| PROPYLENE GLYCOL | 5.000 g |
| MINT FLAVOURING | 0.150g |
| LIQUORICE EXTRACT | 0.100g |
| HYDROGENATED (40) POLYOXYETHYLENATED CASTOR OIL | 0.700 g |
| PONCEAU RED COLOURING E124 | 0.005 g |
| PURIFIED WATER | q.s. to 100 ml |

### Example 4

A composition with the following composition is prepared, according to the procedure of Example 1:

| **INGREDIENTS** | **AMOUNT** |
|---|---|
| DICLOFENAC ACID | 0.074 g |
| CHOLINE BASE 50% | q.s. to pH 7.0-7.5 |
| POLYCARBOPHIL AA1 | 0.1500g |
| CARBOXYPOLYMETHYLENE (CARBOPOL 971 P) | 0.100g |
| XYLITOL | 10.000g |
| SODIUM SACCHARINATE | 0.150g |
| SODIUM EDETATE | 0.025 g |
| SODIUM BENZOATE | 0.500 g |
| GLYCERIN | 5.000 g |
| PROPYLENE GLYCOL | 5.000 g |
| MINT FLAVOURING | 0.150g |
| LIQUORICE DRY EXTRACT | 0.100g |
| HYDROGENATED (40) POLYOXYETHYLENATED CASTOR OIL | 0.700 g |
| PONCEAU RED COLOURING E124 | 0.005 g |
| PURIFIED WATER | q.s. to 100 ml |

### Example 5

A composition with the following composition is prepared, according to the procedure of Example 1:

| **INGREDIENTS** | **AMOUNT** |
|---|---|
| DICLOFENAC SODIUM | 0.150g |
| CHOLINE BASE 50% | q.s. to pH 7.0-7.5 |
| POLYCARBOPHIL (NOVEON AA1) | 0.300 g |
| POLYVINYLPYRROLIDONE K90 | 2.000g |
| XYLITOL | 15.000 g |
| SACCHARINE | 0.120 g |
| SODIUM EDETATE | 0.025 g |
| SODIUM BENZOATE | 0.500g |
| GLYCERIN | 5.000 g |
| PROPYLENE GLYCOL | 5.000g |
| MINT FLAVOURING | 0.150g |
| HYDROGENATED (40) POLYOXYETHYLENATED CASTOR OIL | 0.700 g |
| PONCEAU RED COLOURING E124 | 0.005 g |
| PURIFIED WATER | q.s. to 100 ml |

### Example 6

A composition with the following composition is prepared, according to the procedure of Example 1:

| **INGREDIENTS** | **AMOUNT** |
|---|---|
| DICLOFENAC ACID | 0.222 g |
| CHOLINE BASE 50% | q.s. to pH 7.0-7.5 |
| POLYCARBOPHIL (NOVEONAA1) | 0.025 g |
| POLYVINYLPYRROLIDONE K90 | 5.000 g |
| XYLITOL | 15.000 g |
| ACESULFAME POTASSIUM | 0.100g |
| SODIUM EDETATE | 0.025 g |
| CHLORHEXIDINE DIGLUCONATE | 0.100g |
| GLYCERIN | 5.000 g |
| PROPYLENE GLYCOL | 5.000 g |
| MINT FLAVOURING | 0.150g |
| HYDROGENATED (40) POLYOXYETHYLENATED CASTOR OIL | 0.700 g |
| LIQUORICE DRY EXTRACT | 0.050 g |
| PONCEAU RED COLOURING E124 | 0.005 g |
| PURIFIED WATER | q.s. to 100 ml |

## Claims

1. Bioadhesive pharmaceutical compositions in the form of viscosified solutions or suspensions containing non-steroidal anti-inflammatory drugs, polyvinylpyrrolidone and polyacrylic acids cross-linked with divinyl glycol.

2. Compositions as claimed in claim 1, wherein the non-steroidal anti-inflammatory drug belongs to the class of arylpropionic acids, oxicams, phenylacetic acids or salicylic derivatives.

3. Compositions as claimed in claim 2, wherein the non-steroidal anti-inflammatory drug is diclofenac or a salt thereof.

4. Compositions as claimed in claim 3, wherein the non-steroidal anti-inflammatory drug is diclofenac choline salt.

5. Compositions as claimed in claim 3 or 4, wherein the concentration is between 0.01 % and 1% by weight.

6. Compositions as claimed in any one of the preceding claims, wherein polyvinylpyrrolidone has an average to high molecular weight (K25 - K120) and is present in concentrations of between approx. 1% and approx. 20% on the weight of the total formulation.

7. Compositions as claimed in any one of the preceding claims, wherein the polycarbophil is present in concentrations of between 0.1% and 2% by weight.

8. Compositions as claimed in any one of the preceding claims, further containing sweeteners, flavourings, antioxidants, antimicrobial agents or surfactants.
